Europäisches Patentamt

(19) **European Patent Office**

**Office européen des brevets**

(11) Numéro de publication : **0 058 595**
**B1**

(12) **FASCICULE DE BREVET EUROPÉEN**

(45) Date de publication du fascicule du brevet :
07.08.85

(51) Int. Cl.⁴ : **A 61 K 7/06**

(21) Numéro de dépôt : **82400177.0**

(22) Date de dépôt : **03.02.82**

(54) **Produit et méthode de traitement pour la pousse des cheveux.**

(30) Priorité : **03.02.81 FR 8102010**

(43) Date de publication de la demande :
**25.08.82 Bulletin 82/34**

(45) Mention de la délivrance du brevet :
**07.08.85 Bulletin 85/32**

(84) Etats contractants désignés :
**AT BE CH DE FR GB IT LI LU NL SE**

(56) Documents cités :
**FR-A- 1 021 806**
**FR-A- 2 070 045**
**FR-A- 2 112 112**
**GB-A- 23 922**
**US-A- 1 525 299**
**CHEMICAL ABSTRACTS, vol. 90, no. 22, 28 mai 1979, page 381, no. 174503q, Columbus Ohio (USA); G. HUNGER RICCI et al.: "Cosmetological uses for the essential oil and juice of bergamot"**

(73) Titulaire : **Borrel veuve Alfonsi, Marguerite**
**Bâtiment F-1 Allée des Glaieuls**
**F-91130 Ris Orangis (FR)**

(72) Inventeur : **Borrel veuve Alfonsi, Marguerite**
**Bâtiment F-1 Allée des Glaieuls**
**F-91130 Ris Orangis (FR)**

(74) Mandataire : **Pinguet, André**
**CAPRI 28 bis, avenue Mozart**
**F-75016 Paris (FR)**

Jouve, 18, rue St-Denis, 75001 Paris, France

## Description

La présente invention a trait à des produits de traitement pour assurer au système pileux un développement normal, notamment dans les cas où il se trouve défaillant par exemple par suite d'une maladie, d'une grossesse ou après un traitement agressif, tel qu'une permanente. Les produits selon l'invention conviennent particulièrement aux chevelures féminines. Ils peuvent toutefois être efficaces dans certains cas pour les chevelures masculines.

La présente invention a plus particulièrement pour objet des éléments dont l'association présente des propriétés synergiques inattendues que ne laisse prévoir la simple addition de leurs propriétés particulières.

Après une préparation spéciale du derme, l'application d'une crème conforme à la présente invention assure l'apport des éléments nutritifs nécessaires mais également stimule les processus biologiques de développement ou de redéveloppement du système pileux.

On a déjà proposé de très nombreux produits de traitements de la chute des cheveux mais il semble qu'en pratique, on ne soit pas parvenu jusqu'à présent dans tous les cas à obtenir par des moyens simples et peu coûteux une réelle renaissance du système pileux. Les moyens conformes à la présente invention ne nécessitent ni appareil ou procédés compliqués ni personnel spécialisé.

Dans le brevet français antérieur 2.070.045 déposé par la demanderesse le 31 décembre 1969, et intitulé « Le traitement capillaire et anticalvitie », est proposé un traitement en trois phases faisant appel à un premier nettoyage à l'huile, puis une préparation comprenant du pétrole et enfin un traitement par une crème nourrissante.

Conformément à la présente invention, on obtient des résultats améliorés en appliquant une technique plus simple et grâce surtout à l'emploi d'une préparation sous forme de crème à base de moelle qui va agir au niveau de la kératine et du bulbe, arrêtant la chute des cheveux, stimulant les processus biologiques de développement et assurant la nourriture surtout en cours de croissance et de régénération.

Pour mieux faire comprendre les caractéristiques techniques et les avantages de la présente invention, on va en décrire un exemple de réalisation.

On se référera au cuir chevelu à titre illustratif, mais l'expérience montre que les moyens conformes à l'invention peuvent trouver application à d'autres zones du derme.

On prépare une crème en mélangeant vers 40 °C, dans les proportions préférentielles approximatives suivantes :

1 g d'essence de bergamote
2 g d'alcool d'origine végétale tel qu'un rhum par exemple
92 g de moelle (par exemple de bœuf)
4 g d'huile
1 g de fleurs de soufre.

Des essais de variation des dosages ont permis de définir les limites suivantes pour les différents constituants.

— Essence de bergamote : la proportion doit être comprise entre 0,75 % et 2 %. Une proportion de 0,5 % s'est montrée insuffisante. Avec 2 % on a encore de très bons résultats, mais le produit est un peu agressif. Si la proportion dépasse nettement 2 %, si on met par exemple 5 %, l'essence de bergamote a un effet tel que tous les cheveux tombent et l'on parvient au résultat opposé à celui qui est recherché.

— Alcool végétal : ce sont les extraits de plantes, les essences qui sont par exemple dans le rhum qui sont actifs ; ce n'est pas l'alcool lui-même qui est actif bien que cependant il nettoie et tue les microbes. On peut incorporer jusqu'à 3 ou 4 %. Au-delà, il y a trop d'alcool et le produit devient agressif, compte tenu des autres constituants et du temps d'application. 2 % est très bien toléré. En dessous de 1 %, l'efficacité du produit décroît rapidement.

— Moelle : on utilisera de la moelle de bœuf peu coûteuse et facile à se procurer. Il en faut au moins 90 %. On peut accroître la proportion sans inconvénient, mais cela aurait pour conséquence de réduire les proportions des autres constituants, ce qui affaiblirait l'efficacité du produit.

— Huile : une légère proportion s'est montrée avantageuse. On utilisera entre 2 et 5 %. On peut utiliser soit de la vaseline, soit une huile végétale, telle que l'huile de sésame, d'olive, d'amande douce ou analogue ; soit moitié vaseline, moitié huile. Une plus grande proportion n'est pas nécessaire ; il n'y a pas de danger, mais cela réduirait la proportion de moelle et affaiblirait le rendement.

— Soufre : le soufre aide la formation de la kératine. En dessous de 1 %, il y a une baisse d'efficacité notable. On peut en mettre un peu plus, 2 ou 3 % par exemple. Au-delà de 3 %, il n'y a plus d'amélioration, et le produit devient nocif.

On peut préparer d'abord le cuir chevelu par une application préalable pendant 10 à 25 minutes d'un bain d'argile ; ceci est favorable, mais facultatif au sens de la présente invention. Puis on utilise le nécessaire pour le traitement lui-même. On nettoie soigneusement le cuir chevelu avec un coton ou analogue imbibé d'une lotion d'emploi préalable comprenant environ des parts égales en poids de

2

**0 058 595**

pétrole non désodorisé et d'alcool à 70° dans lequel on a fait préalablement macérer, pendant un mois environ, romarin, thym, clous de girofle, sauge, lavande et fleurs et graines de capucine ou camomille. Cette égalité de proportion entre les deux constituants s'est montrée favorable. Bien entendu on ne sort pas du cadre de la présente invention en s'écartant légèrement de cette formule préférentielle. Puis, après 15 à 20 mn, on applique la crème ci-dessus définie en la faisant bien pénétrer en massant légèrement en directions perpendiculaires pendant 5 minutes. On garde la crème en place au moins 20 minutes et de préférence plusieurs heures si possible (la nuit par exemple) puis on lave et on rince.

Ce nécessaire pour le traitement du cuir chevelu, utilisé une fois par semaine pendant 1 mois, tous les 15 jours le mois suivant, une fois le troisième mois, fait apparaître les cheveux chez la quasi-totalité des sujets, c'est-à-dire après 3 à 8 semaines d'utilisation.

On peut dans la plupart des cas se limiter à 3 mois d'utilisation.

L'expérience a permis de constater que l'association de l'essence de bergamote avec la moelle et autres lipides non seulement stimule la croissance, mais également active la circulation au niveau du derme et assure la nourriture du cheveu. Il est vraisemblable que cet effet est imputable entre autres à l'intervention des psoralènes de la bergamote et en particulier du 5-méthoxy-psoralène.

A titre illustratif, on mentionnera quatre cas dans lesquels on a constaté les résultats suivants :

1. Enfant de 7 ans à cheveux clairsemés : le nécessaire pour le traitement du cuir chevelu tel que défini ci-dessus permet une pousse active et drue de 1,5 cm par mois.

2. Femme 32 ans, cheveux malades, fourchus, ayant tendance à la chute : le nécessaire pour ce traitement du cuir chevelu ci-dessus a redonné aspect et vigueur normale en 7 semaines.

3. Femme de 72 ans, cheveux rares : le traitement ci-dessus a redonné en 2 mois une chevelure à développement normal.

4. Homme de 45 ans, eczéma du derme crânien : le nécessaire pour le traitement ci-dessus a entraîné une desquamation rapide, une régénération du derme et de la kératine, et une repousse normale observée au bout de 9 semaines.

On notera que les compositions définies ci-dessus présentent des effets fongicides et bactéricides remarquables qui constituent des avantages non négligeables.

Par ailleurs, on a observé dans tous les cas une amélioration au niveau du cuir chevelu.

**Revendications**

1. Nécessaire pour le traitement du cuir chevelu caractérisé en ce qu'il comporte au moins le second des deux produits ci-après :

A. Une lotion d'emploi préalable comprenant des proportions sensiblement égales de pétrole non désodorisé et d'alcool à 70° dans lequel on a fait préalablement macérer, pendant un mois environ, romarin, thym, clous de girofle, sauge, lavande et fleurs et graines de capucine ou camomille,

B. Une crème comportant les produits ci-après, dans les proportions suivantes en % en poids :

| | |
|---|---|
| essence bergamote | 0,75 à 2 |
| alcool végétal | 1    à 4 |
| moelle | au moins 90 |
| huile | 2    à 5 |
| fleurs de soufre | 1    à 3 |

2. Nécessaire selon la revendication 1, caractérisé en ce que dans la crème définie en B, les proportions sont sensiblement les suivantes en % en poids :

| | |
|---|---|
| essence bergamote | 1 |
| alcool végétal | 2 |
| moelle | 92 |
| huile | 4 |
| fleurs de soufre | 1 |

3. Nécessaire selon une des revendications 1 ou 2, caractérisé en ce que l'alcool végétal est le rhum.

4. Nécessaire selon une des revendications 1 à 3, caractérisé en ce que l'huile comporte moitié vaseline, moitié huile végétale.

5. Nécessaire selon une des revendications 1 à 4, caractérisé en ce que l'huile est celle de sésame, d'olive ou d'amande.

**Claims**

1. Kit for treating the scalp, characterized in that it comprises at least the second of the following two products :

3

A. A lotion for preliminary use comprising substantially the same amount of non deodorized mineral oil and 70° alcohol, in which have soaked during about one month the following plants : rosemary, thyme, clove, sage, lavender and flowers and seeds of nasturtium and camomile ;

B. A cream comprising the following ingredients with a percentage given in weight :

| | |
|---|---|
| Bergamot oil | 0.75 to 2 |
| Vegetable alcohol | 1 to 4 |
| Marrow | at least 90 |
| Oil | 2 to 5 |
| Flowers of sulphur | 1 to 3 |

2. Kit according to claim 1 wherein the percentages of the cream under B are substantially the following :

| | |
|---|---|
| Bergamot oil | 1 |
| Vegetable alcohol | 2 |
| Marrow | 92 |
| Oil | 4 |
| Flowers of sulphur | 1 |

3. Kit according to claims 1 or 2 wherein the vegetable alcohol is rum.

4. Kit according to one of the claims 1 to 3, wherein the oil comprises one half of a vaseline and one half of vegetable oil.

5. Kit according to one of the claims 1 to 4, wherein the oil is chosen in the group containing the oils of sesame, olive and almond.

**Patentansprüche**

1. Gerät für Kopfhaut Pflege dadurch gekennzeichnet dass es wenigstens das zweite der nachstehenden Produkte einschliesst ;

A. Ein Haarwasser für Vorbenutzung einschliessend ungefähr gleiche Verhältnisse von nicht entgeruchtet Erdöl und 70° alkohol, in dem haben vorerst mazeriert, während ungefähr eines Monates, Rosmarin, Thymian, gewürznelke, Salbei, Lavendel, und Blumen und Samen von Kapuzinerblume oder Kamille ;

B. Eine Creme die die folgende Produkte einschliesst mit der folgenden Verhältnissen, pro Gewicht :

| | |
|---|---|
| Bergamotte Öl | 0,75 bis 2 |
| Pflanze Alkohol | 1 bis 4 |
| Mark | wenigstens 90 |
| Öl | 2 bis 5 |
| Schwefelblumen | 1 bis 3 |

2. Gerät gemäss Anspruch 1, dadurch gekennzeichnet dass, in der in B definierte Creme, die Verhältnisse sind ungefähr die folgende, pro Gewicht :

| | |
|---|---|
| Bergamotte Öl | 1 |
| Pflanze Alkohol | 2 |
| Mark | 92 |
| Öl | 4 |
| Schwefelblumen | 1 |

3. Gerät gemäss Anspruch 1 oder 2, dadurch gekennzeichnet dass das pflanze Alkohol Rum ist.

4. Gerät gemäss einem der Ansprüche 1 bis 3, dadurch gekennzeichnet dass, das Öl eine halbe Vaseli. eine halbe pflanze Öl einschliesst.

5. Gerät gemäss einem der Ansprüche 1 bis 4, dadurch gekennzeichnet, dass das Öl, Sesamöl, Oliveöl oder Mandelöl ist.